(19) Europäisches Patentamt
European Patent Office
Office européen des brevets

(11) Veröffentlichungsnummer: **0 318 704 B1**

(12) # EUROPÄISCHE PATENTSCHRIFT

(45) Veröffentlichungstag der Patentschrift: **02.12.92**

(51) Int. Cl.$^5$: **C07D 207/34**, A01N 43/36

(21) Anmeldenummer: **88118105.1**

(22) Anmeldetag: **31.10.88**

Die Akte enthält technische Angaben, die nach dem Eingang der Anmeldung eingereicht wurden und die nicht in dieser Patentschrift enthalten sind.

(54) **3-Cyano-4-phenyl-pyrrole.**

(30) Priorität: **09.11.87 DE 3737984**

(43) Veröffentlichungstag der Anmeldung:
**07.06.89 Patentblatt 89/23**

(45) Bekanntmachung des Hinweises auf die Patenterteilung:
**02.12.92 Patentblatt 92/49**

(84) Benannte Vertragsstaaten:
**BE CH DE FR GB IT LI NL SE**

(56) Entgegenhaltungen:
**EP-A- 182 738**
**EP-A- 183 217**
**EP-A- 0 236 272**
**DE-A- 2 927 480**
**US-A- 3 590 051**

**Chem. Abs. 95, N. 21, 187069f**

(73) Patentinhaber: **BAYER AG**

**W-5090 Leverkusen 1 Bayerwerk(DE)**

(72) Erfinder: **Wollweber, Detlef, Dr.**
**Paul-Ehrlich-Strasse 17**
**W-5600 Wuppertal 1(DE)**
Erfinder: **Brandes, Wilhelm, Dr.**
**Eichendorffstrasse 3**
**W-5653 Leichlingen(DE)**

**EP 0 318 704 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 3-Cyano-4-phenyl-pyrrole, ein Verfahren zu ihrer Herstellung, ihre Verwendung als Schädlingsbekämpfungsmittel und neue Zwischenprodukte.

Es ist bekannt, daß bestimmte 3-Cyano-4-phenyl-pyrrole wie beispielsweise die Verbindung 3-Cyano-4-(2,3-dichlorphenyl)-pyrrol fungizide Wirksamkeit besitzen (vgl. z. B. EP 236 272 und Chem. Abstr. Vol. 95, Nr. 21, 187 069f), ein Verfahren zur Herstellung von 3-Cyano-4-phenyl-pyrrolen ist beschrieben in EP-A 0 182 738.

Weiterhin gibt es 1-Acetyl-3-cyano-pyrrole mit fungizider Wirkung (vgl. DE 2 927 480), ebenso sind Pyrrolin-Derivate mit mikrobizider Wirksamkeit in EP-A 0 183 217 beschrieben.

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 3-Cyano-4-phenyl-pyrrole der allgemeinen Formel (I),

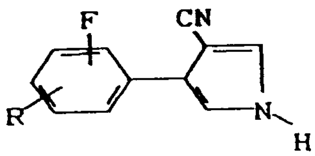 (I)

in welcher

R für Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht,

gefunden.

Weiterhin wurde gefunden, daß man die neuen 3-Cyano-4-phenyl-pyrrole der allgemeinen Formel (I),

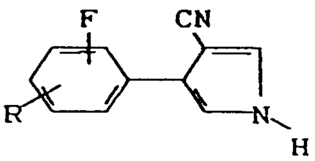 (I)

in welcher

R die oben angegebene Bedeutung hat,

erhält, wenn man substituierte Fluorzimtsäurenitrile der Formel (II),

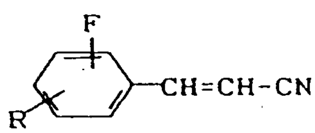 (II)

in welcher

R die oben angegebene Bedeutung hat,

mit Sulfonylmethylisocyaniden der Formel (III),

$R^1-SO_2-CH_2-NC$ (III)

in welcher

$R^1$ für Alkyl oder für gegebenenfalls substituiertes Aryl steht, in Gegenwart einer Base und gegebenenfalls in Gegenwart eines Verdünnungsmittels umsetzt.

Schließlich wurde gefunden, daß die neuen 3-Cyano-4-phenyl-pyrrole der allgemeinen Formel (I) eine gute Wirkung gegen Schädlinge besitzen.

Überraschenderweise zeigen die erfindungsgemäßen 3-Cyano-4-phenyl-pyrrole der allgemeinen Formel (I) eine erheblich bessere fungizide Wirksamkeit als z.B. die aus dem Stand der Technik bekannten 3-Cyano-4-phenyl-pyrrole, wie das 3-Cyano-4-(2,3-dichlor-phenyl)-pyrrol, welches chemisch und wirkungsmäßig naheliegende Verbindung sind.

Die erfindungsgemäßen 3-Cyano-4-phenyl-pyrrole sind durch die Formel (I) allgemein definiert. Bevorzugt sind Verbindungen der Formel (I), bei welchen

R für Fluor, Chlor, Brom, Methyl, Ethyl, Methoxy, Ethoxy, Methylthio, Trifluormethyl, Trifluormethoxy oder Trifluormethylthio steht.

Besonders bevorzugt sind Verbindungen der Formel (I), bei welchen

R für Fluor, Chlor, Methyl, Trifluormethyl oder Trifluormethoxy steht.

Im einzelnen seien außer den bei den Herstellungsbeispielen genannten Verbindungen die folgenden 3-Cyano-4-phenyl-pyrrole der allgemeinen Formel (I) genannt:

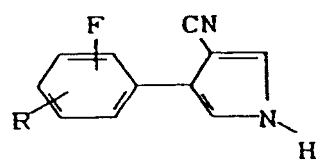

(I)

| 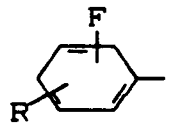 | 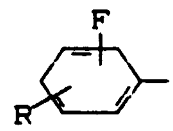 |
| --- | --- |
| 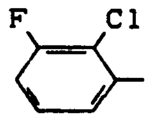 | 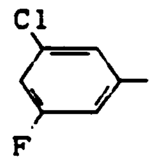 |
| 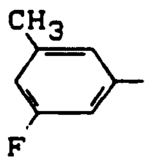 | 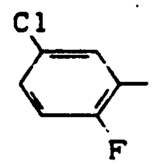 |
| 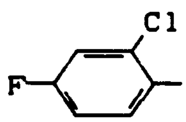 | 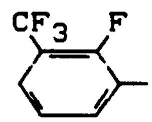 |
| 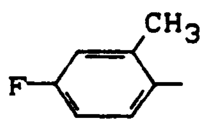 | 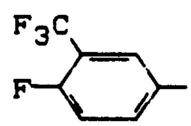 |
| 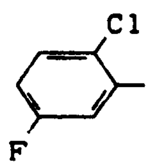 | 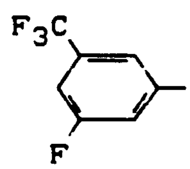 |
| 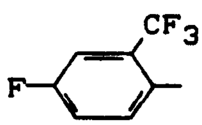 | 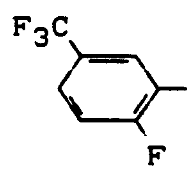 |
| 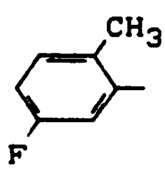 | 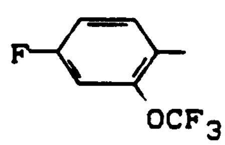 |

| 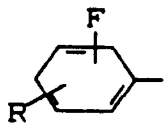 | 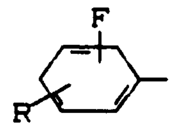 |
| --- | --- |
| 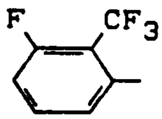 | 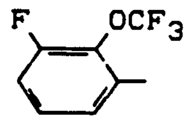 |
| 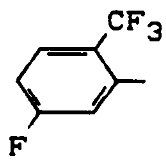 | 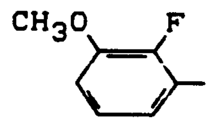 |
| 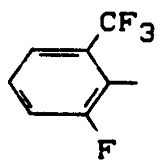 | 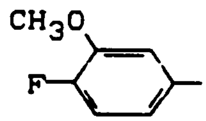 |
| 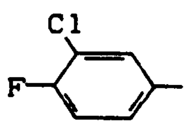 | 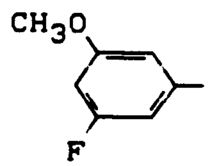 |
| 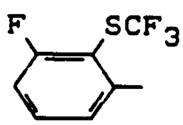 | 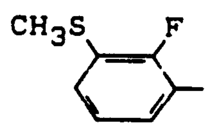 |
| 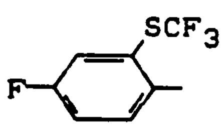 | 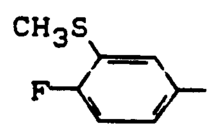 |
| 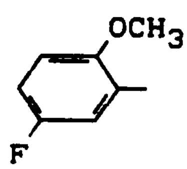 | 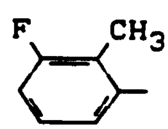 |

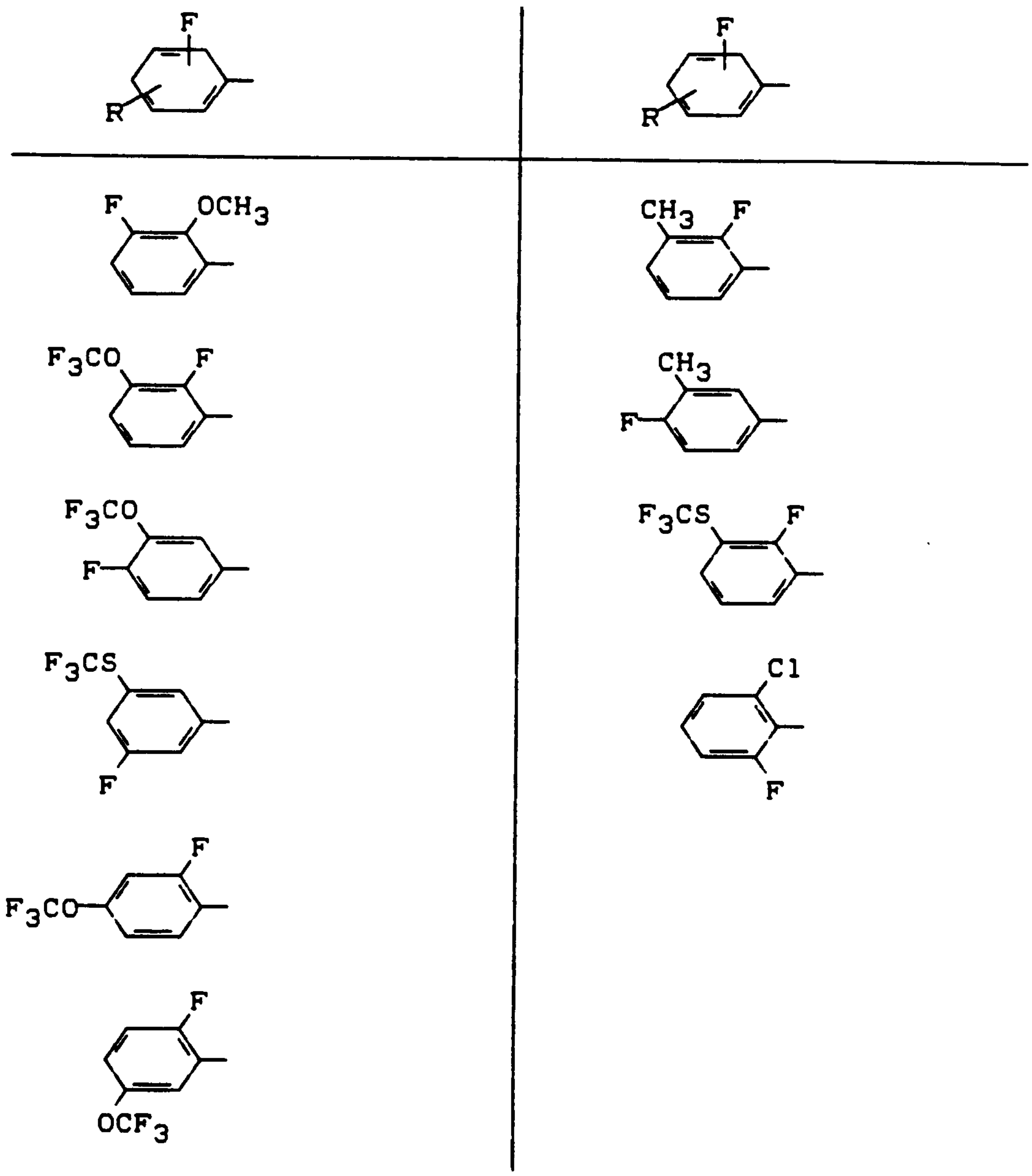

Verwendet man beispielsweise 3-Chlor-2-fluorzimtsäurenitril und p-Toluolsulfonylmethylisocyanid als Ausgangsstoffe und Natriumhydrid als Base, so läßt sich der Reaktionsablauf des erfindungsgemäßen Verfahrens durch das folgende Formelschema darstellen:

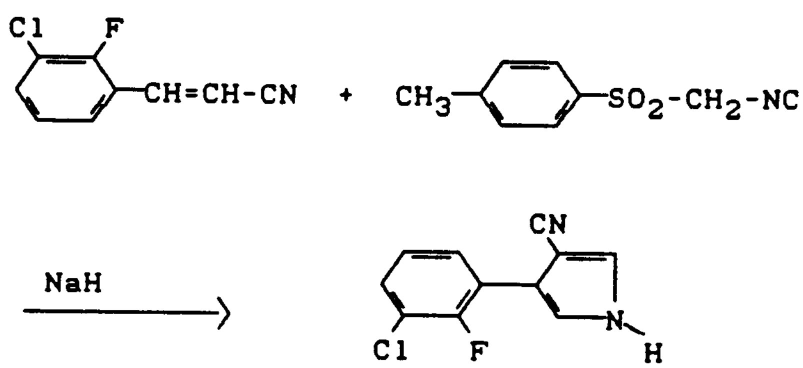

Die zur Durchführung des erfindungsgemäßen Verfahrens als Ausgangsstoffe benötigten substituierten Fluorzimtsäurenitrile sind durch die Formel (II) allgemein definiert. In dieser Formel (II) steht R vorzugsweise für diejenigen Reste, die bereits im Zusammenhang mit der Beschreibung der erfindungsgemäßen Stoffe der Formel (I) als bevorzugt für diesen Substituenten genannt wurden.

Die substituierten Fluorzimtsäurenitrile der Formel (II) sind neu. Man erhält sie jedoch in Analogie zu bekannten Verfahren (vgl. z.B. DE-OS 2 927 480), beispielsweise wenn man

a) Fluoraniline der Formel (IV),

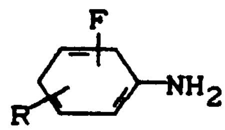

(IV)

in welcher

R die oben angegebene Bedeutung hat,

zunächst in einer ersten Stufe mit Acrylnitril unter üblichen Diazotierungsbedingungen, beispielsweise in Gegenwart von Natriumnitrit und Salzsäure, und in Gegenwart eines geeigneten Metallsalz-Katalysators, wie beispielsweise Kupfer-II-chlorid oder Kupfer-II-oxid, und gegebenenfalls in Gegenwart eines geeigneten Verdünnungsmittels, wie beispielsweise Aceton oder Wasser, bei Temperaturen zwischen - 20 °C und 50 °C umsetzt ("Meerwein-Arylierung"; vgl. hierzu auch Organic Reactions 11, 189 [1960]; Organic Reactions 24, 225 [1976] oder C. Ferri "Reaktionen der organischen Synthese" S. 319, Thieme Verlag Stuttgart 1978) und dann in einer 2. Stufe die so erhältlichen substituierten $\alpha$-Chlor-$\beta$-phenylpropionitrile der Formel (V),

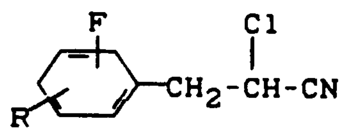

(V)

in welcher

R die oben angegebene Bedeutung hat,

mit Basen, wie beispielsweise Triethylamin oder Diazabicycloundecen, in üblicher Art und Weise gegebenenfalls in Gegenwart eines Verdünnungsmittels, wie beispielsweise Tetrahydrofuran, bei Temperaturen zwischen 0 °C und 50 °C dehydrohalogeniert (vgl. auch die Herstellungsbeispiele) oder alternativ, wenn man

b) Fluorbenzaldehyde der Formel (VI),

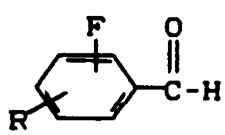

(VI)

in welcher

R die oben angegebene Bedeutung hat,

mit Cyanessigsäure der Formel (VII),

$NC\text{-}CH_2\text{-}COOH$ (VII)

in üblicher Art und Weise in Gegenwart einer Base, wie beispielsweise Piperidin oder Pyridin, und gegebenenfalls in Gegenwart eines geeigneten Verdünnungsmittels, wie beispielsweise Pyridin, bei Temperaturen zwischen 50 °C und 120 °C kondensiert und gleichzeitig decarboxyliert (vgl. z.B. "Organikum" S. 571/572; 15. Auflage; VEB Deutscher Verlag der Wissenschaften Berlin 1981 sowie die Herstellungsbeispiele).

Die Fluoraniline der Formel (IV) sind teilweise bekannt (vgl. z.B. J. org. Chem. 39, 1758-1761 [1974]; J.

med. Chem. 12 195 - 196 [1969] oder US-PS 3 900 519) oder erhältlich in Analogie zu bekannten Verfahren.

Neu sind z.B. Verbindungen der Formel (IVa)

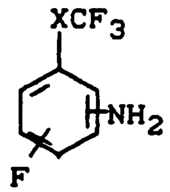

(IVa)

in der

X für Sauerstoff oder Schwefel steht.

Sie sind Gegenstand einer separaten eigenen, nicht zum Stand der Technik gehörenden Patentanmeldung (vgl. deutsche Patentanmeldung P 37 37 985 vom 9. Nov. 1987).

Bevorzugte Verbindungen der Formel (IVa) sind: 2-Fluor-5-amino-fluormethylthio-benzol, 2-Fluor-4-amino-fluormethoxy-benzol, 3-Amino-4-fluor-trifluormethoxy-benzol, 2-Amino-4-fluor-trifluormethoxy-benzol und 2-Amino-5-fluor-trifluormethoxy-benzol.

Ein allgemein anwendbares Verfahren zur Herstellung von Verbindungen der Formel (IVa),

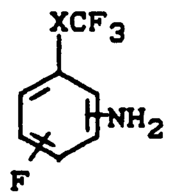

(IVa)

in der

X für Sauerstoff oder Schwefel steht,

ist dadurch gekennzeichnet, daß man Verbindungen der Formel (VIII)

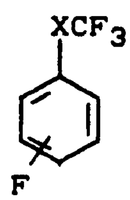

(VIII)

in der

X die bei Formel (IVa) angegebene Bedeutung hat,

nitriert und die so erhaltenen Nitroverbindungen reduziert.

Die in dieses Verfahren einzusetzenden Fluor enthaltenden Trifluormethoxy- und Trifluormethylthiobenzole sind bekannt (J. Org. Chem. 29, 1, 1964).

Die Nitrierung kann mit üblichen Nitriermitteln durchgeführt werden, beispielsweise mit Gemischen aus Salpeter- und Schwefelsäure. Die Temperatur kann dabei beispielsweise im Bereich von 0 bis 80 °C liegen, vorzugsweise liegt sie bei 20 bis 50 °C. Das Nitriermittel kann man beispielsweise in Mengen einsetzen, bei denen im Reaktionsgemisch 0,8 bis 1,5 Mole Nitrieragenz pro Mol Ausgangsverbindung gebildet werden. Vorzugsweise läßt man sich 1 bis 1,1 Mol Nitrieragenz pro Mol Ausgangsverbindung bilden. Die Nitrierung kann gegebenenfalls in Gegenwart eines inerten organischen Lösungsmittels durchgeführt werden. Geeignet ist beispielsweise Methylenchlorid.

Die daran anschließende Reduktion kann chemisch, d.h. beispielsweise mit reduzierend wirkenden

Metallen oder Metallsalzen durchgeführt werden. Geeignet sind beispielsweise Eisen, Zink, Zinn, Zinn(II)-chlorid und Titan(III)chlorid. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder danach isoliert werden. Die Reduktion kann man auch katalytisch mit Wasserstoff durchführen, wobei z.B. Katalysatoren eingesetzt werden können, die Metalle enthalten oder daraus bestehen. Geeignet sind beispielsweise die Metalle der VIII. Nebengruppe des Periodischen Systems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder aufgebracht als Metall oder Metallverbindung auf Trägermaterialien. Bevorzugt ist Raney-Nickel und Palladium auf Kohle und Aluminiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran. Die katalytische Reduktion kann beispielsweise bei Temperaturen wie im präparativen Beispiel angegeben und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Gegebenenfalls sind letztere also von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile abfiltriert und das Filtrat, gegebenenfalls nach einer Wäsche mit Wasser, destilliert. Falls als Reaktionsprodukt ein Gemisch von Isomeren anfällt, kann man dieses durch Feindestillation trennen.

Verbindungen der Formel (IVa) mit Fluor in o- oder p-Stellung zur Aminogruppe kann man auch herstellen, indem man Verbindungen der Formel (IX),

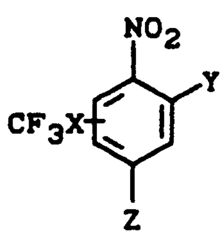

(IX)

in der

X für Sauerstoff oder Schwefel und
mindestens eines der Symbole Y und Z für Chlor und das andere für Wasserstoff steht,

mit Fluorierungsmitteln wie Kaliumfluorid in Gegenwart von polaren, aprotischen Lösungsmitteln wie Tetramethylensulfon umsetzt, wobei vorhandenes Chlor durch Fluor ersetzt wird, und anschließend eine Reduktion durchführt, wobei die Nitrogruppe in eine Aminogruppe überführt wird.

Verbindungen der Formel (IX) sind bekannt (vgl. Houben Weyl, Methoden der organischen Chemie E4, Seite 633 ff.)

Tetramethylensulfon fungiert als Lösungsmittel und wird vorzugsweise mindestens in einer solchen Menge eingesetzt, daß ein gut rührbares Reaktionsgemisch vorliegt. Größere Mengen an Tetramethylensulfon stören nicht.

Geeignete Temperaturen für die Umsetzung mit Kaliumfluorid in Tetramethylensulfon sind beispielsweise solche im Bereich von 160 bis 230 °C. Bevorzugte Temperaturen sind solche von 180 bis 210 °C. Vorzugsweise führt man die Umsetzung in möglichst wasserfreiem Milieu durch. Dies kann man beispielsweise dadurch erreichen, daß man als letzte Komponente die Verbindung der Formel (IX) in sorgfältig getrockneter Form einsetzt und aus den vorher zusammengegebenen sonstigen Komponenten eine kleine Menge Tetramethylensulfon zusammen mit gegebenenfalls vorhandenem Wasser abdestilliert.

Nach Beendigung der Reaktion können im Reaktionsgemisch vorliegende Feststoffe und gegebenenfalls ganz oder teilweise das Tetramethylensulfon entfernt werden.

Die anschließende Reduktion der Nitro- zur Aminogruppe und die Aufarbeitung des dann vorliegenden Reaktionsgemisches kann so erfolgen, wie zuvor beim allgemein anwendbaren Verfahren zur Herstellung der Verbindungen beschrieben worden ist.

Verbindungen der Formel (IVa) mit X = Sauerstoff können auch hergestellt werden, indem man Verbindungen der Form (X)

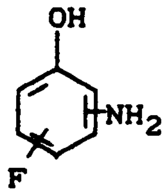

(X)

mit Tetrachlorkohlenstoff in Gegenwart von Fluorwasserstoff umsetzt, wobei die OH-Gruppe in eine $CF_3O$-Gruppe überführt wird.

Verbindungen der Formel (X) sind bekannt (vgl. FR 2 446 805).

Pro Mol der jeweiligen Verbindung der Formel (X) kann man beispielsweise 1 bis 10 Mol Tetrachlorkohlenstoff und 5 bis 30 Mol Fluorwasserstoff einsetzen. Auch größere Überschüsse von Tetrachlorkohlenstoff und Fluorwasserstoff stören im allgemeinen nicht. Geeignete Reaktionstemperaturen sind beispielsweise solche im Bereich von 100 bis 150 ° C. Dieses Verfahren führt man vorzugsweise unter Druck durch, beispielsweise indem man das entstehende Chlorwasserstoffgas erst oberhalb eines bestimmten Druckes entspannt. Dieser Druck kann beispielsweise bei 18 bis 60 bar liegen. Gegebenenfalls kann man zusätzlich ein inertes Gas aufdrücken, z.B. 1 bis 20 bar Stickstoff. Es ist vorteilhaft, während der Reaktion gut zu rühren.

Die Aufarbeitung des Reaktionsgemisches kann man beispielsweise so durchführen, daß man das Reaktionsgemisch auf Raumtemperatur abkühlt, entspannt, überschüssigen Fluorwasserstoff und überschüssigen Tetrachlorkohlenstoff beispielsweise bei Temperaturen bis 80 ° C abdestilliert, den Rückstand auf Eiswasser gibt, mit Natronlauge alkalisch stellt, die organische Phase mit Dichlormethan extrahiert und nach dem Trocknen einer Feindestillation unterwirft.

Weiterhin sind Verbindungen der Formel (IVb),

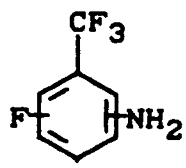

(IVb)

bei denen sich die Trifluormethylgruppe in 1-Stellung, die Aminogruppe
a) in 2-Stellung
und ein Fluoratom in 6-Stellung oder
b) in 3-Stellung
und ein Fluoratom in 2-Stellung
befinden, neu.

Sie sind Gegenstand einer nicht zum Stand der Technik gehörenden eigenen Anmeldung (vgl. deutsche Patentanmeldung P 37 37 986 vom 9. Nov. 1987).

Ein bevorzugtes Verfahren zur Herstellung Fluor enthaltender Trifluormethylaminobenzole, bei denen sich die Trifluormethylgruppe in 1-Stellung, die Aminogruppe
a) in 2-Stellung
und ein Fluoratom in 6-Stellung oder
b) in 3-Stellung
und ein Fluoratom in 2-Stellung
befinden, ist dadurch gekennzeichnet, daß man entsprechende, Fluor enthaltende Trifluormethylbenzole nitriert und die so erhaltenen Fluor enthaltenden Trifluormethylnitrobenzole reduziert.

Die in dieses erfindungsgemäße Verfahren einzusetzenden Fluor enthaltenden, aber von Aminogruppen freien Trifluormethylbenzole sind bekannt. Die Nitrierung, die anschließende Reduktion und Aufarbeitung erfolgt bei den zur Herstellung von Verbindungen der Formel (IVa) angegebenen Bedingungen.

Ein weiteres Verfahren speziell zur Herstellung von Fluor enthaltenden Trifluormethylaminobenzolen, bei denen sich die Trifluormethylgruppe in 1-Stellung, die Aminogruppe
a') in 2-Stellung und ein Fluoratom in 6-Stellung oder

b') in 4-Stellung und ein Fluoratom in 2-Stellung

befinden, ist dadurch gekennzeichnet, daß man entsprechende Fluor enthaltende, 2- und/oder 4-Halogen-trifluormethylbenzole bei erhöhtem Druck mit Ammoniak in Gegenwart eines organischen Lösungsmittels umsetzt.

Die in dieses Verfahren einzusetzenden 2- und/oder 4-Halogen-trifluormethylbenzole sind bekannt.

Das Ammoniak kann in flüssiger oder gasförmiger Form zugesetzt werden, z.B. in Substanz (gasförmig oder flüssig) oder als wäßrige Lösung. Pro Mol in 2- und/oder 4-Stellung durch $NH_2$-Gruppen zu ersetzende Halogenatome kann man beispielsweise 1 bis 10 Mol Ammoniak verwenden. Bevorzugt beträgt diese Menge 3 bis 8 Mol. Geeignete Temperaturen für diese Umsetzung sind beispielsweise solche im Bereich von 80 bis 160 °C, bevorzugt sind solche im Bereich 100 bis 130 °C. Die Umsetzung kann unter dem sich im geschlossenen Gefäß bei Reaktionstemperatur einstellenden Eigendruck des Ammoniaks durchgeführt werden, der beispielsweise im Bereich von 10 bis 20 bar liegen kann. Man kann auch höhere Drucke anwenden, beispielsweise solche bis zu 100 bar.

Als Lösungsmittel für diese Umsetzung sind inerte oder weitgehend inerte organische Lösungsmittel der verschiedensten Art einsetzbar. Beispielsweise kommen in Frage: Alkohole, Ether, Sulfone und aromatische Kohlenwasserstoffe.

Aus dem nach der Umsetzung vorliegenden Reaktionsgemisch kann man das oder die gewünschten Reaktionsprodukte z.B. erhalten, indem man zunächst abkühlt und den Druck entspannt, dann das Lösungsmittel entfernt und anschließend eine Destillation, vorzugsweise unter vermindertem Druck, durchführt.

Die weiterhin als Vorprodukte zur Herstellung der neuen Ausgangsprodukte der Formel (II) nach Variante b) benötigten Fluorbenzaldehyde der Formel (VI) sind größtenteils bekannt (vgl. z.B. Chem. Abstr. 100; 209 388k = Jap. Pat. 58/222 045), so ist der 5-Fluor-2-trifluormethyl-benzaldehyd mit der Reg.-Nr. 90 381-08-1 in Chem. Abstr., der 3-Chlor-5-fluor-benzaldehyd mit 90 390-49-1, der 3-Chlor-6-fluor-benzaldehyd mit 96 515-79-6 usw. genannt. Die Cyanessigsäure der Formel (VII) ist ebenfalls eine allgemein bekannte Verbindung der organischen Chemie.

Die zur Durchführung des erfindungsgemäßen Verfahrens weiterhin als Ausgangsstoffe benötigten Sulfonylmethylisocyanide sind durch die Formel (III) allgemein definiert. In dieser Formel (III) steht $R^1$ vorzugsweise für Methyl oder für gegebenenfalls einfach substituiertes Phenyl, wie biespielsweise 4-Methylphenyl, 4-Chlorphenyl oder Phenyl.

Die Sulfonylmethylisocyanide der Formel (III) sind bekannt (vgl. z.B., Synthesis 1985, 400-402; Org. Syntheses 57, 102-106 [1977]; J. org. Chem. 42, 1153-1159 [1977]; Tetrahedron Lett. 1972, 2367-2368).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid, oder Sulfoxide, wie Dimethylsufloxid.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen - 30 °C und + 120 °C, vorzugsweise bei Temperaturen zwischen - 20 °C und + 50 °C.

Zur Durchführung des erfindungsgemäßen Verfahrens setzt man pro Mol an substituiertem Fluorzimtsäurenitril der Formel (II) im allgemeinen 1.0 bis 2.0 Mol, vorzugsweise 1.0 bis 1.5 Mol an Sulfonylmethylisocyanid der Formel (III) und 1.0 bis 2.0 Mol, vorzugsweise 1.0 bis 1.5 Mol an Base ein.
Dabei ist es gegebenenfalls von Vorteil, die Reaktion in Gegenwart einer Schutzgasatmosphäre wie beispielsweise Argon durchzuführen.
Die Reaktionsdurchführung, Aufarbeitung und Isolierung der Reaktionsprodukte erfolgt nach allgemein üblichen Methoden.

Alternativ zur Herstellung der erfindungsgemäßen Wirkstoffe mit Hilfe des erfindungsgemäßen Herstellungsverfahrens sind verschiedene weitere Herstellungsverfahren zur Herstellung der erfindungsgemäßen Wirkstoffe denkbar.

So erhält man erfindungsgemäße Wirkstoffe der Formel (I) beispielsweise auch, wenn man α-Cyanozimtsäureester in Gegenwart von Basen und in Gegenwart von Kupfer-(II)-Salzen mit p-Toluolsulfonylmethylisocyanid umsetzt (vgl. J6-1030-571 oder J6-1200-984) oder wenn man α-substituierte Zimtsäurenitrile in Gegenwart von Natriumhydrid mit Isocyanoessigsäureestern cyclisiert, die so erhältlichen Pyrrol-2-carbonsäureester mit Basen verseift und anschließend thermisch decarboxyliert (vgl. JP 59/212 468) oder wenn man Phenacylaminderivate mit geeignet substituierten Acrylnitril-Derivaten umsetzt (vgl. EP 174 910) oder wenn man 3-Trifluormethyl-4-phenyl-pyrrole mit Ammoniak bei erhöhter Temperatur und erhöhtem Druck umsetzt (vgl. EP 182 738) oder wenn man 3-Cyano-4-phenyl-$\Delta^2$-pyrroline in Gegenwart von Kupfer-II-Salzen oder Eisen-III-salzen oxidiert (vgl. EP 183 217) oder wenn man α-Cyanoacrylsäurederivate mit Isocyanoessigsäureestern in Gegenwart einer Base umsetzt und die so erhältlichen $\Delta^2$-Pyrrolin-2-carbonsäurederivate in einer 2. Stufe in Gegenwart einer Base und in Gegenwart eines Metallsalzkatalysators oxidativ decarboxyliert (vgl. Deutsche Patentanmeldung P 3 718 375 vom 02.06.1987).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von

Krankheiten im Obst- und Gemüseanbau, wie beispielsweise gegen den Erreger des Bohnengrauschimmels (Botrytis cinerea) oder zur Bekämpfung von Reiskrankheiten wie beispielsweise gegen den Erreger der Reisfleckenkrankheit (Pyricularia oryzae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylene-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

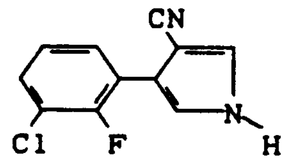

Zu 1,4 g (0,0464 Mol) Natriumhydrid (80 %ig in Mineralöl) in 17,5 ml Tetrahydrofuran unter einer Argonschutzgasatmosphäre gibt man bei - 10 ° C bis - 20 ° C tropfenweise unter Rühren eine Lösung von 6,0 g (0.0331 Mol) 3-(2-Fluor-3-chlor-phenyl)-acrylnitril und 7,8 g (0.0431 Mol) p-Toluolsulfonylmethylisocyanid in 20 ml einer Mischung aus Tetrahydrofuran/Dimethylsulfoxid (5:1). Nach beendeter Zugabe läßt man die Reaktionsmischung auf Raumtemperatur kommen, gibt Wasser zu, extrahiert mehrfach mit Essigester, wäscht die vereinigten Essigesterphasen mit Wasser, trocknet über Natriumsulfat und engt im Vakuum ein. Der Rückstand wird durch Chromatographie an Kieselgel (Laufmittel: Cyclohexan/Essigester 5:1) gereinigt.

Man erhält 3,3 g (45 % der Theorie) an 3-Cyano-4-(2-fluor-3-chlorphenyl)-pyrrol vom Schmelzpunkt 180 ° C - 181 ° C.

In entsprechender Weise und gemäß den allgemeinen Angaben zur Herstellung erhält man die folgenden 3-Cyano-4-phenyl-pyrrole der allgemeinen Formel (I):

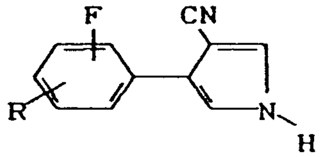

(I)

| Bsp.Nr. | 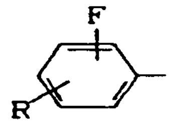 | Schmelzpunkt[° C] |
|---|---|---|
| 2 | 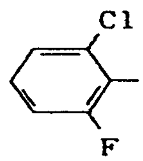 | 126 - 128 |
| 3 | 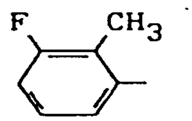 | 133 - 134 |
| 4 | 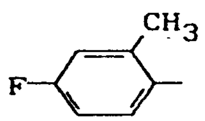 | 130 - 131 |
| 5 | 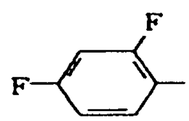 | 176-177 |
| 6 | 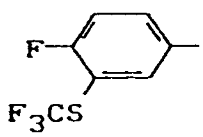 | 160-161 |

Herstellung der Ausgangsverbindungen

Beispiel II-1

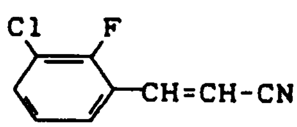

Zu einer Lösung aus 40,1 g (0.25 Mol) 2-Fluor-3-chlor-benzaldehyd in 170 ml Pyridin gibt man 2,5 ml Piperidin und 22,9 g (0.27 Mol) Cyanessigsäure und erhitzt 14 Stunden auf Rückflußtemperatur. Zur Aufarbeitung engt man im Vakuum ein, nimmt den Rückstand in Essigester auf, wäscht nacheinander mit 1 normaler Salzsäure, mit wässriger Natriumhydrogensulfitlösung sowie mit Wasser, trocknet über Natriumsulfat und engt im Vakuum ein. Das verbleibende Öl läßt sich durch Chromatographie an Kieselgel (Laufmittel: Cyclohexan/Essigester 5:1) reinigen.

Man erhält 16,9 g (37 % der Theorie) an 3-(2-Fluor-3-chlor-phenyl)-acrylnitril vom Schmelzpunkt 90 °C - 92 °C.

Beispiel II-2

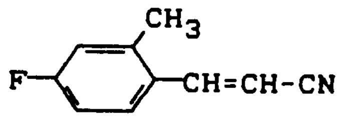

Zu 21,7 g (0.11 Mol) 2-Chlor-3-(4-fluor-2-methyl-phenyl)-propionitrilin 100 ml Tetrahydrofuran tropft man bei Raumtemperatur unter Rühren 18,2 g (0,12 Mol) Diazabicycloundecen in 150 ml Tetrahydrofuran, rührt nach beendeter Zugabe 15 Stunden bei Raumtemperatur, filtriert, engt das Filtrat im Vakuum ein, nimmt den Rückstand in Essigester auf, wäscht nacheinander mit 1 normaler Salzsäure und Wasser, trocknet über Natriumsulfat und entfernt das Lösungsmittel im Vakuum.

Man erhält 34,6 g (96 % der Theorie) an 3-(4-Fluor-2-methyl-phenyl)-acrylnitril vom Schmelzpunkt 86 °C - 87 °C.

In entsprechender Weise und gemäß den allgemeinen Angaben zur Herstellung erhält man die folgenden Fluorzimtsäurenitrile der allgemeinen Formel (II):

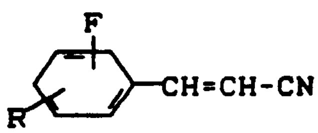

(II)

| Bsp.Nr. | 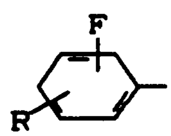 | physikalische Eigenschaften |
|---|---|---|
| II-3 | 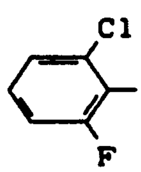 | Fp. 59 - 60 °C |
| II-4 | 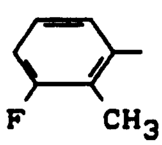 | Fp. 76 - 77 °C |

Beispiel (IV-1)

a)

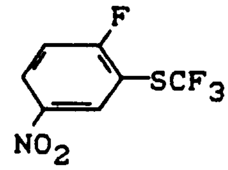

In 100 ml Tetramethylensulfon werden 20 g Kaliumfluorid suspendiert und bei 15 mbar ca. 20 ml Lösungsmittel abdestilliert. Dann werden 30 g 3-Trifluormethylmercapto-4-chlor-nitrobenzol zugegeben und für 6 Stunden bei 190 °C unter Feuchtigkeitsausschluß gerührt. Nach dem Abkühlen des Gemisches auf 25 °C wird es auf 300 ml Wasser gegossen und mehrmals mit Toluol extrahiert. Die Toluolphase wird zweimal mit Wasser gewaschen, dann getrocknet und destilliert. Bei einem Siedepunkt von 115 bis 120 °C bei 18 mbar gehen 20 g 2-Fluor-5-nitro-trifluormethylthio-benzol über.

b)

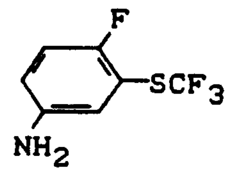

In einer Rührapparatur werden 20 g der gemäß a) hergestellten Nitroverbindung mit 10 ml Dioxan, 150 ml Wasser, 17 g Eisenpulver und 2 g Ammoniumchlorid vorgelegt und dann für 5 Stunden am Rückfluß erhitzt. Die abgekühlte Lösung wird filtriert und der Filterrückstand mehrmals mit Dichlormethan gewaschen. Nach Extraktion des Produktes aus der wäßrigen Phase mittels Dichlormethan werden die vereinigten organischen Phasen getrocknet und vom Lösungsmittel befreit. Die Feindestillation des Produktes liefert 14 g 2-Fluor-5-amino-trifluormethylthio-benzol mit einem Siedepunkt von 112 bis 114 °C bei 16 mbar.

Beispiel IV-2

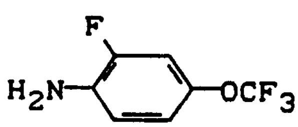

In einer Fluorierungsapparatur werden 100 g 2-Fluor-4-hydroxy-anilin, 500 ml wasserfreier Fluorwasserstoff und 500 ml Tetrachlormethan vorgelegt und 3 bar Stickstoff aufgedrückt. Anschließend wird unter Rühren mit 400 U/min für 7 Stunden auf 120 °C erhitzt und der entstehende Chlorwasserstoff bei 38 bar kontinuierlich entspannt. Nach Ende der Reaktion wird das Gemisch auf 20 °C gekühlt, entspannt und überschüssiger Fluorwasserstoff zusammen mit Tetrachlorkohlenstoff bis 80 °C abdestilliert. Der Rückstand wird nach Abkühlen auf 500 ml Eiswasser gegossen und unter Kühlung mit Natronlauge alkalisch gestellt. Anschließend wird die organische Phase mit Dichlormethan extrahiert, getrocknet und im Vakuum einer Feindestillation unterworfen. Bei einem Siedepunkt von 68 bis 69 °C bei 20 mbar gehen 53 g 3-Fluor-4-amino-trifluormethoxy-benzol über.

Beispiel IV-3

a)

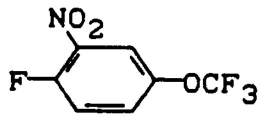

In einer Rührapparatur werden 90 g 4-Trifluormethoxyfluorbenzol vorgelegt und bei 10 bis 15 °C 105 g Mischsäure (33 Gew.-% Salpetersäure, 67 Gew.-% Schwefelsäure) zugetropft. Es wird für 2 Stunden bei 20 °C nachgerührt und dann auf Eis gegossen. Nach Abtrennung der organischen Phase mit Hilfe von Dichlormethan wird die Lösung getrocknet und destilliert. Im Siedebereich von 90 bis 94 °C bei 15 mbar geht ein Gemisch bestehend aus 43 Gew.-% 3-Nitro-4-fluor-trifluormethoxy-benzol und 54 Gew.-% 2-Nitro-4-fluor-trifluormethoxy-benzol in einer Menge von 112 g über.

b)

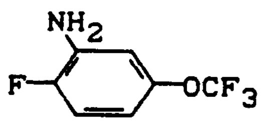

In einer Hydrierapparatur werden 112 g der gemäß a) erhaltenen Nitroverbindungen in 450 ml Methanol zusammen mit 12 g Raney-Nickel vorgelegt und mit 30 bar Wasserstoff bei 25 bis 45 °C hydriert. Nach Abkühlen und Entspannen wird das Gemisch filtriert und anschließend das Filtrat einer Feindestillation unterworfen. Im Siedebereich 58 bis 60 °C bei 14 mbar gingen 32 g 2-Amino-4-fluor-trifluormethoxy-benzol über und nach einem Zwischenlauf bei 64 bis 65 °C und 15 mbar 28 g 3-Amino-4-fluor-trifluormethoxy-benzol.

Beispiel IV-4

a)

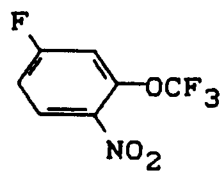

Analog Beispiel IV-3 werden 83 g 3-Trifluormethoxyfluorbenzol mit 100 g Nitriersäure bei 15 °C nitriert. Die Reaktionsmischung besteht nach analoger Aufarbeitung zu 78 Gew.-% aus 3-Fluor-4-amino-trifluormethoxy-benzol und zu 20 Gew.-% aus 3-Fluor-6-nitro-trifluormethoxy-benzol. Die Gesamtausbeute betrug 99 g.

b)

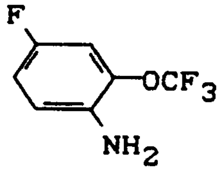

In einer Hydrierapparatur werden die gemäß a) hergestellten 99 g Nitroverbindungen in 350 ml Methanol in Gegenwart von 10 g Raney-Nickel bei 25 bis 40 °C mit 30 bis 50 bar Wasserstoff hydriert. Nach Ende der Wasserstoffaufnahme wird abgekühlt und entspannt, die festen Bestandteile des Reaktionsgemisches abfiltriert und danach das Lösungsmittel durch Destillation entfernt. Die Feindestillation des

Rückstandes lieferte im Siedebereich von 53 bis 54 °C bei 10 mbar 5 g 3-Fluor-6-amino-trifluormethoxy-benzol und nach einem Zwischenlauf 38 g 3-Fluor-4-amino-trifluormethoxy-benzol in einem Siedebereich von 56 bis 57 ° C bei 10 mbar.

Beispiel IV-5

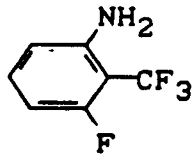

In einem Edelstahlautoklaven werden 100 g 2,6-Difluorbenzotrifluorid in 300 ml Tetrahydrofuran vorgelegt und 30 ml flüssiger Ammoniak aufgedrückt. Nach stufenweisem Aufheizen auf 125 °C wird bei dieser Temperatur für 5 Stunden nachgerührt. Nach Abkühlen auf 20 °C wird entspannt und die Lösung unter vermindertem Druck destilliert. Bei einem Siedepunkt von 49 bis 50 °C bei 8 mbar gehen 46 g 2-Amino-6-fluor-benzotrifluorid über.

Beispiel IV-6

a) 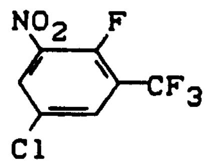

In einer Rührapparatur werden 100 g 2-Fluor-5-chlorbenzotrifluorid vorgelegt und 120 g Nitriersäure (33 Gew.-% Salpetersäure und 67 Gew.-% Schwefelsäure) bei 40 °C zugetropft. Es wird noch für eine Stunde bei 40 bis 45 °C nachgerührt, dann abgekühlt und auf Eis gegossen. Die organische Phase wird mit Dichlormethan extrahiert, getrocknet und destilliert. Bei einem Siedepunkt von 92 bis 94 °C bei 18 mbar gehen 112 g Nitroverbindungen über, die zu 91,3 Gew.-% 2-Fluor-5-chlor-3-nitro-benzotrifluorid enthalten.

b) 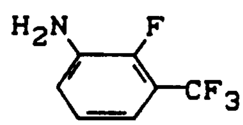

In einer Hydrierapparatur werden 111 g der gemäß a) erhaltenen Nitroverbindungen in 500 ml Tetrahydrofuran vorgelegt und nacheinander 50 g Triethylamin und 15 g Raney-Nickel zugegeben. Nach Spülen der Apparatur mit Wasserstoff wird bei 25 bis 120 °C mit einem Wasserstoffdruck von 30 bis 80 bar hydriert. Nach Ende der Wasserstoffaufnahme wird abgekühlt und entspannt. Das Reaktionsgemisch wurde filtriert, mit Tetrahydrofuran nachgewaschen und aus den vereinigten Filtraten das Tetrahydrofuran bei Normaldruck zum größten Teil über eine Kolonne abdestilliert. Der Sumpf wurde mit 200 ml Wasser verrührt, dann die organische Phase abgetrennt, getrocknet und unter vermindertem Druck einer Feindestillation unterworfen. Bei einem Siedepunkt von 70 bis 72 °C bei 16 mbar gingen 52 g 2-Fluor-3-amino-benzotrifluorid über.

Beispiel V-1

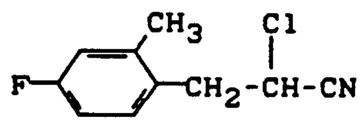

Zu 15 g (0.12 Mol) 4-Fluor-2-methylanilin in 40 ml Aceton gibt man 40 ml 25prozentige Salzsäure und 28,8 ml (0.37 Mol) Acrylsäurenitril, tropft dann bei 0 °C bis 10 °C unter Rühren innerhalb einer Stunde 8,7 g (0,13 Mol) Natriumnitrit in 17 ml Wasser zu, rührt eine weitere Stunde bei 0 °C bis 10 °C und gibt dann mehrere Portionen Kupfer-II-oxid-Pulver zu, wobei eine heftige Stickstoffgasentwicklung zu beobachten ist. Nach beendeter Gasentwicklung rührt man weitere 15 Stunden bei Raumtemperatur, gibt dann Dichlormethan zu, wäscht mit Wasser, trocknet über Natriumsulfat, engt im Vakuum ein und reinigt den öligen Rückstand durch Chromatographie an Kieselgel (Laufmittel: Cyclohexan/Essigester 1:1).

Man erhält 44,2 g (89,3 % der Theorie) an 2-Chlor-3-(4-fluor-2-methylphenyl)-propionitril als Öl.

$^1$H-NMR* ($CDCl_3$/TMS : $\delta$ = 3,3 (2 H); 6,9 (2 H); 4,5 (1 H); 7,2 (1 H); 2,4 (3 H) ppm.

In entsprechender Weise erhält man Beispiel V-2:

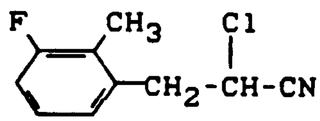

$^1$H-NMR* $CDCl_3$/TMS : $\delta$ = 2,3 (3 H); 3,35 (2 H); 4,5 (1 H); 7,0 (2 H); 7,15 (1 H) ppm.

*) Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Anwendungsbeispiel

In dem folgenden Anwendungsbeispiel wurde die nachstehend aufgeführte Verbindung als Vergleichssubstanz eingesetzt:

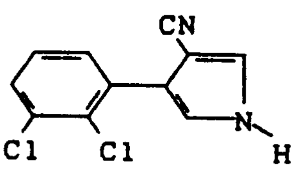

(A)

3-Cyano-4-(2,3-dichlorphenyl)-pyrrol
(vgl. EP 174 910 sowie EP 236 272).

Beispiel A

Botrytis-Test (Bohne)/protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß Herstellungsbeispielen 1 und 5.

Beispiel B

Leptosphaeria nodorum-Test (Weizen) / protektiv

| | |
|---|---|
| Lösungsmittel: | 100 Gew.-Teile Dimethylformamid |
| Emulgator: | 0,25 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Knokulation erfolgt die Auswertung.

Eine deutliche Überlegenheit in der Wirksamkeit gegenüber dem Stand der Technik zeigt bei diesem Test z.B. die Verbindungen gemäß dem Herstellungsbeispiel 1.

Beispiel C

Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10°C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigt bei diesem Test z.B. die Verbindungen gemäß dem Herstellungsbeispiel 1.

## Patentansprüche

**1.** 3-Cyano-4-phenyl-pyrrole der allgemeinen Formel (I)

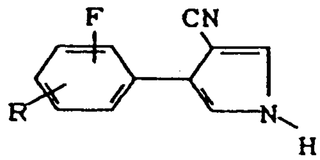

(I)

in welcher

R für Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht.

**2.** 3-Cyano-4-phenyl-pyrrole gemäß Anspruch 1, wobei in der Formel (I)

R für Fluor, Chlor, Brom, Methyl, Ethyl, Methoxy, Ethoxy, Methylthio, Trifluormethyl, Trifluormethoxy oder Trifluormethylthio steht.

**3.** 3-Cyano-4-phenyl-pyrrole gemäß Anspruch 1, wobei in der Formel (I)

R für Fluor, Chlor, Methyl, Trifluormethyl oder Trifluormethoxy steht.

**4.** Verfahren zur Herstellung von 3-Cyano-4-phenyl-pyrrolen der allgemeinen Formel (I) gemäß Anspruch 1, dadurch gekennzeichnet, daß man substituierte Fluorzimtsäurenitrile der Formel (II),

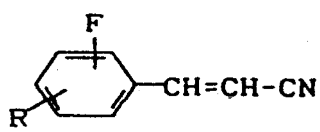

(II)

in welcher

R die in Anspruch angegebene Bedeutung hat,

mit Sulfonylmethylisocyaniden dar Formel (III),

$R^1-SO_2-CH_2-NC$ (III)

in welcher

$R^1$ für Alkyl oder für gegebenenfalls substituiertes Aryl steht, in Gegenwart einer Base und gegebenenfalls in Gegenwart eines Verdünnungsmittels umsetzt.

**5.** Schädlingsbekämpfungsmittel, gekennzeichnet durch einen Gehalt an mindestens einem 3-Cyano-4-phenyl-pyrrol der Formel (I) nach den Ansprüchen 1 und 4.

**6.** Verwendung von 3-Cyano-4-phenyl-pyrrolen der Formel (I) nach den Ansprüchen 1 und 4 zur Bekämpfung von Schädlingen.

**7.** Verfahren zur Bekämpfung von Schädlingen, dadurch gekennzeichnet, daß man 3-Cyano-4-phenyl-pyrrole der Formel (I) nach den Ansprüchen 1 und 4 auf Schädlinge und/oder ihren Lebensraum einwirken läßt.

**8.** Verfahren zur Herstellung von Schädlingsbekämpfungsmitteln, dadurch gekennzeichnet, daß man 3-Cyano-4-phenyl-pyrrole der Formel (I) nach den Ansprüchen 1 und 4 mit Streckmitteln und/oder oberflächenaktiven Mitteln vermischt.

**9.** Substituierte Fluorzimtsäurenitrile der allgemeinen Formel (II)

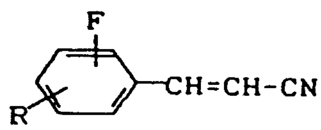

(II)

in welcher

R die in Anspruch 1 angegebene Bedeutung hat

**10.** Verfahren zur Herstellung von substituierten Fluorzimtsäurenitrilen der allgemeinen Formel (II) gemäß Anspruch 9 dadurch gekennzeichnet, daß man

a) Fluoraniline der Formel (IV),

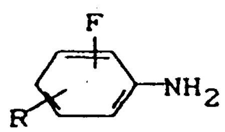

(IV)

zunächst in einer ersten Stufe mit Acrylnitril unter üblichen Diazotierungsbedingungen und in Gegenwart eines geeigneten Metallsalz-Katalysators und gegebenenfalls in Gegenwart eines geeigneten Verdünnungsmittels bei Temperaturen zwischen -20 °C und 50 °C umsetzt und dann in einer 2. Stufe die so erhältlichen substituierten α-Chlor-β-phenyl-propionitrile der Formel (V),

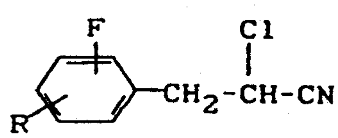

(V)

in welchen

R die in Anspruch 9 angegebene Bedeutung hat,

mit Basen gegebenenfalls in Gegenwart eines Verdünnungsmittels bei Temperaturen zwischen 0 °C und 50 °C dehydrohalogeniert oder

b) Fluorbenzaldehyde der Formel (VI),

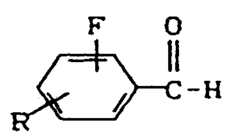

(VI)

in welcher

R die in Anspruch 9 angegebene Bedeutung hat,

mit Cyanessigsäure der Formel (VII)

$NC-CH_2-COOH$ (VII)

in Gegenwart einer Base, gegebenenfalls in Gegenwart eines geeigneten Verdünnungsmittels, bei Temperaturen zwischen 50 °C und 120 °C kondensiert und gleichzeitig decarboxyliert.

**Claims**

**1.** 3-Cyano-4-phenyl-pyrroles of the general formula (I)

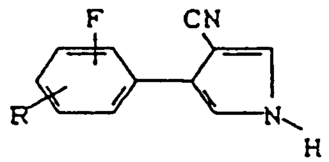

(I)

in which

R represents fluorine, chlorine, bromine, iodine, in each case straight-chain or branched alkyl, alkoxy or alkylthio, each having 1 to 4 carbon atoms, or represents in each case straight-chain or branched halogenoalkyl, halogenoalkoxy or halogenoalkylthio, each having 1 to 4 carbon atoms and 1 to 9 identical or different halogen atoms.

**2.** 3-Cyano-4-phenyl-pyrroles according to Claim 1, where in the formula (I)

R represents fluorine, chlorine, bromine, methyl, ethyl, methoxy, ethoxy, methylthio, trifluoromethyl, trifluoromethoxy or trifluoromethylthio.

**3.** 3-Cyano-4-phenyl-pyrroles according to Claim 1, where in the formula (I)

R represents fluorine, chlorine, methyl, trifluoromethyl or trifluoromethoxy.

**4.** Process for the preparation of 3-cyano-4-phenyl-pyrroles of the general formula (I) according to Claim 1, characterized in that substituted fluorocinnamonitriles of the formula (II)

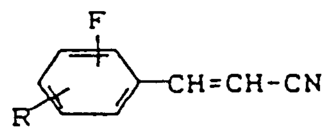

(II)

in which

R has meaning specified in Claim 1,

are reacted with sulphonylmethyl isocyanides of the formula (III)

$R^1-SO_2-CH_2-NC$ (III)

in which

$R^1$ represents alkyl or optionally substituted aryl, in the presence of a base and if appropriate in the presence of a diluent.

**5.** Pesticides, characterized in that they contain at least one 3-cyano-4-phenyl-pyrrole of the formula (I) according to Claims 1 and 4.

**6.** Use of 3-cyano-4-phenyl-pyrroles of the formula (I) according to Claims 1 and 4 for combating pests.

**7.** Method of combating pests, characterized in that 3-cyano-4-phenyl-pyrroles of the formula (I) according to Claims 1 and 4 are allowed to act on the pests and/or their environment.

**8.** Process for the preparation of pesticides, characterized in that 3-cyano-4-phenyl-pyrroles of the formula (I) according to Claims 1 and 4 are mixed with extenders and/or surfactants.

**9.** Substituted fluorocinnamonitriles of the general formula (II)

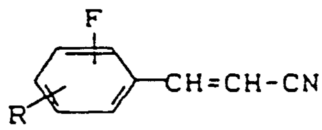

(II)

in which

R has the meaning specified in Claim 1.

**10.** Process for the preparation of substituted fluorocinnamonitriles of the general formula (II) according to Claim 9, characterized in that

a) fluoroanilines of the formula (IV)

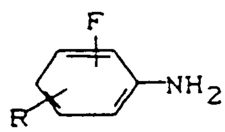

(IV)

are, initially in a first step, reacted with acrylonitrile under the customary conditions for diazotization, and in the presence of a suitable metal salt catalyst and if appropriate in the presence of a suitable diluent, at temperatures between -20 ° C and 50 ° C and then, in a 2nd step, the substituted α-chloro-β-phenyl-propionitriles of the formula (V) thus obtainable

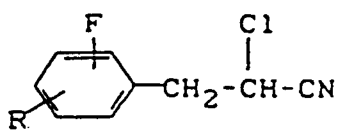

(V)

in which

R has the meaning specified in Claim 9,

are dehydrohalogenated with bases, at temperatures between 0 ° C and 50 ° C, if appropriate in the presence of a diluent, or

b) fluorobenzaldehydes of the formula (VI)

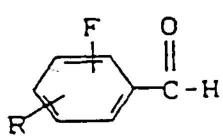

(VI)

in which

R has the meaning specified in Claim 9,

and cyanoacetic acid of the formula (VII)

$NC-CH_2-COOH$ (VII)

are subjected to a condensation reaction at temperatures between 50 ° C and 120 ° C, in the presence of a base, and if appropriate in the presence of a suitable diluent, with simultaneous decarboxylation.

## Revendications

**1.** 3-cyano-4-phényl-pyrroles répondant à la formule générale (I) :

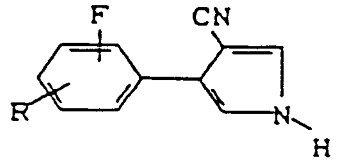

(I)

dans laquelle

R représente un atome de fluor, un atome de chlore, un atome de brome, un atome d'iode, un groupe alkyle, un groupe alcoxy ou un groupe alkylthio, chacun à chaîne droite ou ramifiée et contenant chacun de 1 à 4 atomes de carbone, ou encore un groupe halogénalkyle, un groupe halogénalcoxy ou un groupe halogénalkylthio, chacun à chaîne droite ou ramifiée et contenant chacun de 1 à 4 atomes de carbone et de 1 à 9 atomes d'halogène identiques ou différents.

**2.** 3-cyano-4-phényl-pyrroles selon la revendication 1, dans lesquels, dans la formule (I) :

R représente un atome de fluor, un atome de chlore, un atome de brome, un groupe méthyle, un groupe éthyle, un groupe méthoxy, un groupe éthoxy, un groupe méthylthio, un groupe trifluorométhyle, un groupe trifluorométhoxy ou un groupe trifluorométhylthio.

**3.** 3-cyano-4-phényl-pyrroles selon la revendication 1, dans lesquels, dans la formule (I) :

R représente un atome de fluor, un atome de chlore, un groupe méthyle, un groupe trifluorométhyle ou un groupe trifluorométhoxy.

**4.** Procédé pour la préparation de 3-cyano-4-phényl-pyrroles, répondant à la formule générale (I) selon la revendication 1, caractérisé en ce qu'on fait réagir des nitriles substitués de l'acide fluorocinnamique de formule (II) :

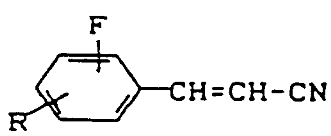

(II)

dans laquelle

R a la signification indiquée à la revendication 1,

avec des sulfonylméthylisocyanures de formule (III) :

$R^1-SO_2-CH_2-NC$ (III)

dans laquelle

$R^1$ représente un groupe alkyle ou un groupe aryle éventuellement substitué, en présence d'une base et éventuellement en présence d'un diluant.

**5.** Agent de lutte contre les parasites, qui se caractérise par une teneur en au moins un 3-cyano-4-phényl-pyrrole de formule (I) selon les revendications 1 et 4.

**6.** Utilisation des 3-cyano-4-phényl-pyrroles de formule (I) selon les revendications 1 et 4, pour lutter contre les parasites.

**7.** Procédé pour lutter contre les parasites, caractérisé en ce qu'on laisse agir des 3-cyano-4-phényl-pyrroles de formule (I) selon les revendications 1 et 4, sur des parasites et/ou sur leur biotope.

**8.** Procédé pour la préparation d'agents de lutte contre les parasites, caractérisé en ce qu'on mélange des 3-cyano-4-phényl-pyrroles de formule (I) selon les revendications 1 et 4, avec des diluants et/ou des agents tensioactifs.

**9.** Nitriles substitués de l'acide fluorocinnamique, répondant à la formule générale (II) :

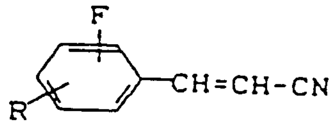

(II)

dans laquelle
R a la signification indiquée dans la revendication 1.

**10.** Procédé pour la préparation de nitriles substitués de l'acide fluorocinnamique répondant à la formule (II), selon la revendication 9, caractérisé en ce qu'on fait réagir

a) des fluoroanilines de formule (IV)

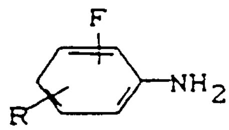

(IV)

dans laquelle
R a la signification mentionnée ci-dessus,
d'abord dans une première étape, avec de l'acrylonitrile dans des conditions habituelles de diazotation et en présence d'un catalyseur approprié de sel métallique et éventuellement en présence d'un diluant approprié à des températures entre -20°C et 50°C, et ensuite, dans une deuxième étape, on fait subir une déshydrohalogénation aux α-chloro-β-phénylpropionitriles substitués que l'on obtient ainsi et qui répondent à la formule (V)

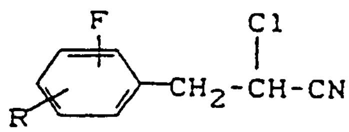

(V)

dans laquelle
R a la signification indiquée dans la revendication 9,
avec des bases, éventuellement en présence d'un diluant à des températures entre 0°C et 50°C ou bien

b) on condense des fluorobenzaldéhydes de formule (VI)

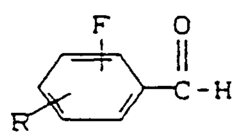

(VI)

dans laquelle
R a la signification indiquée à la revendication 9,
avec de l'acide cyanoacétique de formule (VII)

$NC-CH_2-COOH$ (VII)

en présence d'une base, éventuellement en présence d'un diluant approprié, à des températures

entre 50 ° C et 120 ° C, et on procède simultanément à une décarboxylation.